# EUROPEAN PATENT SPECIFICATION

(11) **EP 3 061 498 B1**
(45) Date of publication and mention of the grant of the patent: **22.11.2017**
(21) Application number: 16155012.4
(22) Date of filing: 10.02.2016
(51) Int. Cl.: A61N 5/10, A61B 34/30, A61G 13/02, A61G 13/04, A61B 6/04

(54) **DEVICE FOR SUPPORTING AND POSITIONING A PATIENT IN A MEDICAL EQUIPMENT**
VORRICHTUNG ZUM STÜTZEN UND POSITIONIEREN EINES PATIENTEN IN EINER MEDIZINISCHEN VORRICHTUNG
DISPOSITIF DE SUPPORT ET DE POSITIONNEMENT D'UN PATIENT DANS UN ÉQUIPEMENT MÉDICAL

(30) Priority: 24.02.2015 LU 92662
(43) Date of publication of application: 31.08.2016
(73) Proprietor: Ion Beam Applications S.A., 1348 Louvain-la-Neuve (BE)
(72) Inventor: DEBATTY, Alexandre, 1348 Louvain-la-Neuve (BE); WIKLER, David, 1348 Louvain-la-Neuve (BE); DOUBLIEZ, Paul-François, 1348 Louvain-la-Neuve (BE)
(74) Representative: Pronovem

(56) References cited:
- DE-A1-102013 005 982
- US-A1- 2004 172 757
- US-A1- 2005 129 181
- US-A1- 2009 184 260

## Description

### Technical Field

The present invention generally relates to a device for positioning a patient in a medical equipment, in particular a radiation therapy equipment. It relates more particularly to a device for supporting and positioning a patient in a medical equipment (in particular a radiation therapy equipment) comprising a patient support means, a robotic arm supporting the patient support means and an orientation mechanism (or robotic wrist) with at least two motorized rotational degrees of freedom, wherein the robotic wrist is borne by the robotic arm and bears the patient support means.

### Background Art

Such a device is also called "patient positioning system (PPS)". In a radiation therapy equipment, the patient positioning system has to warrant a very precise positioning and orientation of the patient, who is supported on a patient support table. Therefore, a modern patient positioning system for such equipment includes a robotic arm and an orientation mechanism (also called robotic wrist), wherein the orientation mechanism is borne by the robotic arm and bears the patient support means. The robotic arm basically allows to position the patient support table in space, by adjusting the X,Y,Z coordinates of the centre of the patient support table. The robotic wrist generally has three rotational degrees of freedom allowing a back and forward tilting, a top rotation and a rolling movement of the patient support table. All these degrees of freedom are principally motorized using drive means with a very high reduction ratio to achieve a slow motorized motion (for safety reasons) and a very precise positioning.

In the final irradiation position, the patient to be irradiated is sandwiched between an irradiation nozzle and the patient support table. There are situations in which it is required to bring the patient rapidly out of this "sandwiched position", for example, if the patient suddenly suffers a seizure, a respiratory failure or any other problem, or simply for temporarily allowing better access to the patient or to a specific body part of the patient, or if there is any technical failure in the medical equipment or in the patient positioning system. Using the motorized degrees of freedom patient positioning system for this purpose has the disadvantage of being rather slow, and this is of course not a solution if there is a failure in the patient positioning system itself. Furthermore, if an emergency stop button is pushed when the patient is in the afore-described "sandwiched position", then all motorized movements are principally disabled, and the patient will remain blocked in this "sandwiched position" until the system gets restarted.

To be able to release the patient manually in such situations, most prior art patient positioning systems provide the possibility to manually actuate the drive means of at least one motorized degree of freedom of the patient positioning system with a dedicated tool, as for example a special crank lever. However, because of the very high reduction ratio in the drive means, this manual actuation of the drive means is very slow. For example, in some prior art patient positioning systems, more than thousand rotations of a crank lever are required to manually release a patient. Furthermore, the operators have to be regularly trained to be capable of efficiently using such a dedicated tool for manually releasing the patient, and it must of course be warranted that the dedicated tool is immediately at hand. It will also be appreciated that a manual actuation of the drive means will generally require a new axis zeroing of the patient positioning system, before being able to reuse the patient positioning system in normal operation. Last but not least, because of the rather complicated mechanics and kinematics of the robotic wrist, it is very complicated to act on the latter for releasing the patient out of its sandwiched therapy position.

An object of the present invention is to simplify release of a patient in patient positioning system equipped with a robotic arm and a robotic wrist.

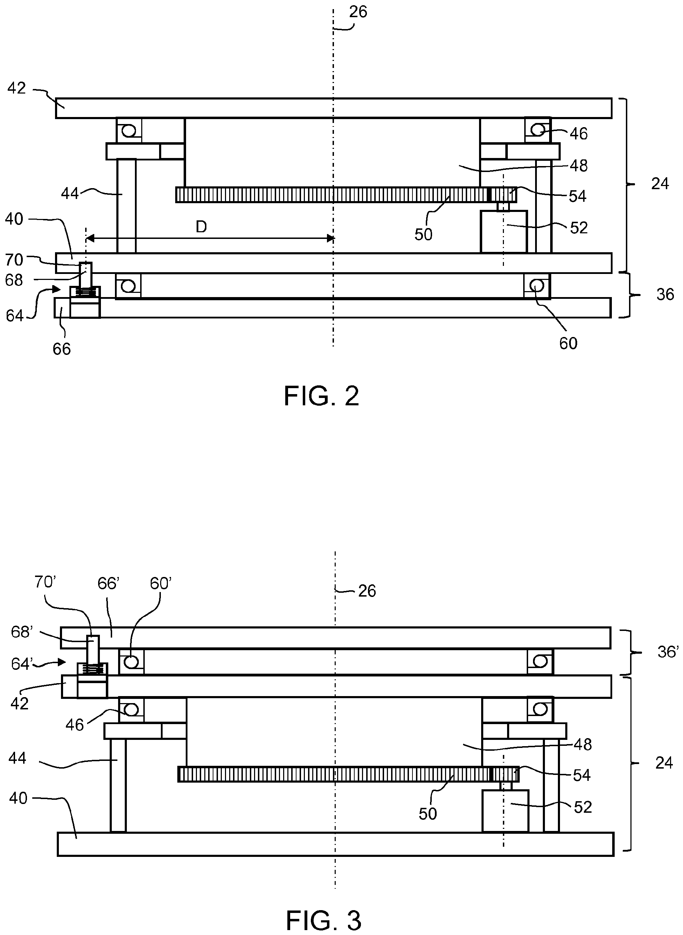

US 2005/129181 discloses a diagnostic table for a medical imaging apparatus in which the patient support table is supported by a supporting unit conceived as a compact pedestal having, according to Fig. 3A and 3B, three translational degrees of freedom B, C, D and two rotational degrees of freedom A, E. A control system with magnetic brakes and clutches allows a controlled floating and panning of the patient support table. This document does obviously not disclose a robotic arm with a robotic wrist.

US 2004/172757 discloses a multiconfiguration braking system in a patient positioning system. In this patient positioning system, the patient support surface is also support by a compact pedestal comprising a telescopic lift system, a longitudinal system, a tilt system, a lateral system, and a rotation system. The rotation system is arranged at the base of the pedestal and may be used for more easily loading and unloading the patient. The multiconfiguration braking system allows to block and release the rotation system. This document too does obviously not disclose a robotic arm with a robotic wrist.

### Summary of invention

The present invention concerns more particularly a device for supporting and positioning a patient in a medical equipment, comprising: a patient support means; a robotic arm supporting the patient support means; and an orientation mechanism (or robotic wrist) with at least two motorized rotational degrees of freedom, the orientation mechanism being borne by the robotic arm and bearing the patient support means. According to a first aspect of the invention, a translational release unit is connected between the orientation mechanism (or robotic wrist) and the patient support means. This translational release unit includes: an XY translation mechanism providing two translational degrees of freedom; and a translation locking mechanism cooperating with the XY translation mechanism. The translation locking mechanism is switchable between a locked state, in which it locks the two translational degrees of freedom of the XY translation mechanism in a mechanically defined position, and an unlocked state, in which the two translational degrees of freedom are unlocked. It will be appreciated that in the unlocked state of the translation locking mechanism, the translational release unit allows to manually release the patient by simply pulling and/or pushing directly on the patient support means. The at least two motorized rotational degrees of freedom of the orientation mechanism (or robotic wrist) remain unaffected, so that the operator is exclusively confronted with a simple translational movement for freeing the patient. With this system, it becomes for example possible to manually push and/or pull the patient support means temporarily in a position allowing a better access to the patient or to a specific body part of the patient, and to push and/or pull it, thereafter, manually back into its therapy position, which corresponds to the mechanically defined position of the XY translation mechanism in its locked state. It will be appreciated that a new axis zeroing of the orientation mechanism (or robotic wrist) is generally not required after such an operation.

Each degree of freedom is for example embodied by a linear stage, comprising a platform and a base, which are joined by a linear guide or bearing means, in such a way that the platform is restricted to guided linear motion with respect to the base.

Preferably, each stage comprises a separate translation locking mechanism.

In a preferred embodiment, the translation locking mechanism comprises a locking pin providing in its locked state a form-locked locking in said mechanically defined position.

The locking pin is advantageously a tapered pin, which is capable of engaging a tapered guide hole, so as to provide, in said mechanically defined position, an auto-centring function in the direction of the translational degree of freedom to be blocked.

In a preferred embodiment, the rotation locking mechanism has to be powered to switch into the locked state and, if it is unpowered, it switches into the unlocked state. Thus it becomes possible to release the patient even if there is no power for operating the rotation locking mechanism.

The translation locking mechanism preferably includes a linear drive for driving a locking member in a locking position. The linear drive is electrically, hydraulically or pneumatically powered, and it advantageously includes a passive element, preferably a spring, for urging the locking member out of the locking position, if the linear drive is unpowered.

A preferred embodiment of the rotation locking mechanism includes a pneumatic cylinder with a cylinder chamber, a piston, a piston rod and a return spring. The return spring retracts the piston rod into the cylinder chamber when the latter is vented. A control valve is connected to the cylinder chamber. This control valve connects the cylinder chamber to a pressure source when it valve is powered, and it vents the cylinder chamber, when it is unpowered.

The switching of the translation locking mechanism from the locked state into the unlocked state is advantageously triggered by simultaneously pushing two release buttons, which are preferably arranged so that an operator has to use his two hands to trigger this switching.

In a preferred embodiment, the orientation mechanism (or robotic wrist) includes three motorized rotational degrees of freedom, for controlling: a pitch angle, which allows a back and forward tilting of the patient support table; a top rotation angle, which allows a planar swivelling of the patient support table; and a roll angle, which allows a side to side pivoting of the patient support table. In this case, the two translational degrees of freedom of the translation release unit are advantageously parallel to a plane that is perpendicular to the axis of the top rotation angle.

The XY translation mechanism is advantageously centred on the axis of the top rotation angle, when it is in its locked state. With regard to its centred position, the XY translation mechanism advantageously provides a degree of freedom of +/- x according to the X-axis and of +/- y according to the Y axis, wherein the absolute values of x and y are both in the range of 300 mm to 800 mm.

In a preferred embodiment, the robotic arm includes at least one motorized rotary joint member for positioning the patient support means by means of a motorized pivoting motion about a pivot axis. A rotational release unit is in this case advantageously associated with the motorized rotary joint member. This rotational release unit comprises: an override bearing arranged adjacent to or in the in the motorized rotary joint member so as to be substantially coaxial with the pivot axis, and to allow a free pivoting motion of the positioning mechanism about the pivot axis; and a rotation locking mechanism cooperating with the override bearing, the rotation locking mechanism being switchable between a locked state, in which it locks the override bearing in a mechanically defined angular position, and an unlocked state, in which the override bearing is unlocked and the positioning mechanism can freely pivot about the pivot axis.

The afore-described robotic arm advantageously comprises two motorized rotary joint members defining two substantially vertical pivot axes, wherein each of the two motorized rotary joint members has associated therewith a rotational release unit as specified in the previous paragraph.

The robotic arm may also comprise a motorized rotary joint member that has a substantially horizontal pivot axis and, associated therewith, a rotational release unit. In this case, a damper or brake is advantageously integrated in the rotational release unit for slowing down a gravity caused pivoting motion about the substantially horizontal pivot axis, when the rotation locking mechanism of the rotational release unit is switched from the locked state into the unlocked state.

### Brief description of drawings

The afore-described and other features, aspects and advantages of the invention will be better understood with regard to the following description of illustrative embodiments of the invention and upon reference to the attached drawings, wherein:
- FIG. 1:: is a schematic elevation view showing a device for supporting and positioning a patient in a medical equipment;
- FIG. 2:: is a schematic section of a first embodiment of a motorized rotary joint member with an associated rotational release unit;
- FIG. 3:: is a schematic section of a second embodiment of a motorized rotary joint member with an associated rotational release unit;
- FIG. 4:: is a schematic section of a third embodiment of a motorized rotary joint member with an associated rotational release unit;
- FIG. 5:: is a schematic section of a fourth embodiment of a motorized rotary joint member with an associated rotational release unit;
- FIG. 6:: is perspective view of an XY-translational release unit;
- FIG. 7A:: is a schematic diagram illustrating a locking mechanism of the release unit in a locked state;
- FIG. 7B:: is a schematic diagram illustrating the locking mechanism of the release unit of FIG. 7A in a unlocked state; and
- FIG. 8:: is a schematic diagram showing a locking pin and a coopering recess of the locking mechanism of the release unit.

### Detailed description of preferred embodiments

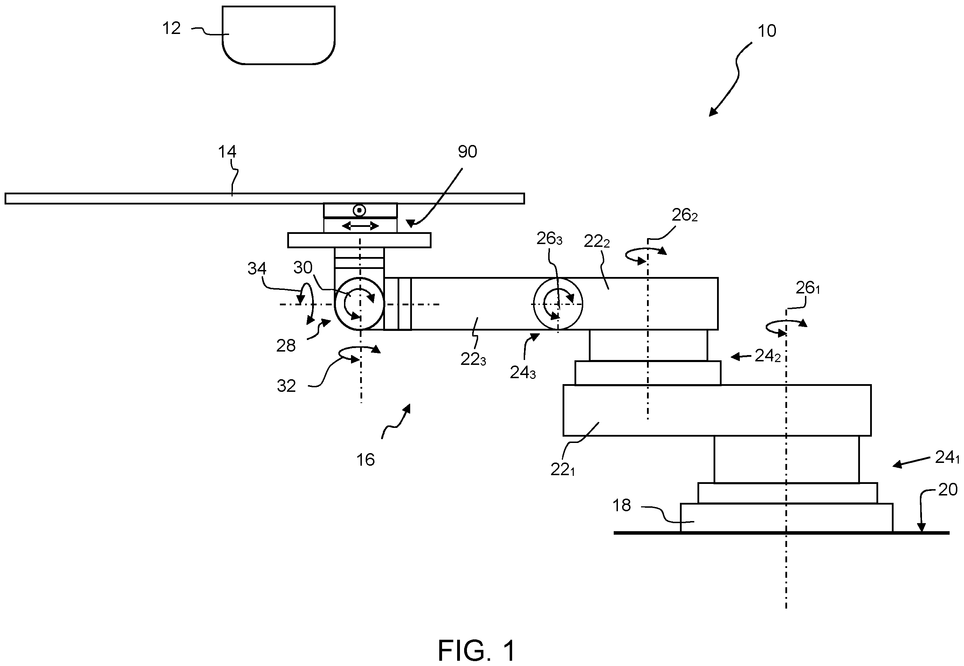

FIG. 1 schematically shows a device 10 for supporting and positioning a patient in a medical equipment, which is e.g. a radio therapy equipment schematically represented by an irradiation nozzle 12. It will however be appreciated that the device 10 can also be used for supporting and positioning a patient in other medical equipment. In general, a device in accordance with the present invention is of advantage, if a very precise motorized positioning of a patient in a treatment position is required, but there is simultaneously a need to bring this patient rapidly out of this treatment position.

The device 10 shown in FIG. 1 includes a patient support means 14, which is normally a patient support table, also called patient couch, but it could also be a treatment chair or the like. In FIG. 1, this patient support table 14 is located below the irradiation nozzle 12. A patient to be irradiated (not shown) lies on the patient couch 14, sandwiched between the irradiation nozzle 12 and the patient couch 14. It will be noted that there are many situations in which it will be required to bring the patient rapidly out of this "sandwiched position".

The patient support table 14 is supported by a positioning mechanism 16, here a robotic arm, which is itself supported by means of a support base 18 on a floor 20, in a pit or on any kind of external support structure. In the present case, the robotic arm 16 comprises three arm members 22₁, 22₂, 22₃ (generally referred to as support members). The first arm member 22₁ is connected to the support base 18 by means of a first motorized rotary joint member 24₁, which allows a motorized pivoting motion of the first arm member 22₁ relatively to the support base 18 and about a first pivot axis 26₁, which is substantially vertical. The second arm member 22₂ is connected to the first arm member 22₁ by means of a second motorized rotary joint member 24₂, which allows a motorized pivoting motion of the second arm member 22₂ relatively to the first arm member 22₁ and about a second pivot axis 26₂, which is substantially parallel to the first pivot axis 26₁ (i.e. the second pivot axis 26₂ is vertical too). The first arm member 22₁ and the second arm member 22₂ allow to adjust, in a known manner, the horizontal X, Y coordinates of the patient support means 14. The third arm member 22₃ is connected to the second arm member 22₂ by means of a third motorized rotary joint member 24₃, which allows a motorized pivoting motion of the third arm member 22₃ relatively to the second arm member 22₂ and about a third pivot axis 26₃, which is substantially horizontal. This third arm member 22₃ allows a raising or lowering of the patient support means 14, i.e. to adjust, also in a known manner, the vertical Z-coordinate of the patient support means 14. In an alternative embodiment, the robotic arm includes for example, also in a known manner, a vertical translational degree of freedom, for adjusting the vertical Z-coordinate of the patient support means 14, and two rotational degrees of freedom about two parallel vertical axis, for adjusting the horizontal X, Y coordinates of the patient support means 14.

The robotic arm 16 supports the patient support table 14 by means of an orientation mechanism 28, which is also called "robotic wrist". This orientation mechanism (or robotic wrist) 28 allows to adjust the orientation of the patient support table 14 according to three rotational degrees of freedom, which are called: pitch angle 30 (allowing a back and forward tilting of the patient support table 14), top rotation angle 32 (allowing a planar swivelling of the patient support table 14), and roll angle 34 (allowing a side to side pivoting of the patient support table 14).

FIG. 2 schematically illustrates the mechanical layout of a first embodiment of a motorized rotary joint member 24 with a rotational release unit 36. The motorized rotary joint member 24 extends between a first flange 40 and a second flange 42. The first flange 40 bears a spacing structure 44. The second flange 42 is connected to the spacing structure 44 of the first flange 40 by means of a joint bearing 46. The latter defines an axis of rotation forming the pivot axis 26 of the final motorized rotary joint member 24, i.e. the axis about which a motorized pivoting motion of the support member 22 connect to the second flange 42 will take place.

Reference 48 identifies a tubular drive shaft, which is supported by the second flange 42, and which supports an annular drive gear 50 coaxially with the pivot axis 26. A motor unit 52 is fixed on the first flange 40 and includes a pinion 54, which meshes with the annular drive gear 50 for pivoting the second flange 42 about the pivot axis 26. If the pivoting motion is limited to an angle of less than 360°, the annular drive gear 50 too may be an annular drive gear segment of less than 360°. The motor unit 52 generally comprises an electric motor and a gearbox designed to achieve slow motorized pivoting motion and a very precise angular positioning. As a consequence of the very high reduction ratio, which is due to the gearbox and to the large diameter annular drive gear 50 (a typical diameter of this drive gear would be in the range of 300 mm to 800 mm) cooperating with the relatively small diameter pinion 54, it will hardly be possible to rotate by hand any arm member 22 connected to the second flange 42.

Associated with the motorized rotary joint member 24 is a rotational release unit 36. The latter mainly comprises an override bearing 60 and a rotation locking mechanism 64 cooperating with the override bearing 60. The override bearing 60 is arranged axially adjacent to the rotary joint member 24, so as to be substantially coaxial with the pivot axis 26. In FIG. 2, the override bearing 60 is connected between an auxiliary flange 66 and the first flange 40 of the motorized rotary joint member 24.

The rotation locking mechanism 64 is switchable between a locked state, in which it locks the override bearing 60 in rotation in a mechanically defined angular position, and an unlocked state, in which the override bearing 60 is unlocked, so that the first flange 40 can freely rotate relative to the auxiliary flange 66. In FIG. 2, this rotation locking mechanism 64 is supported by the auxiliary flange 66 and includes a locking pin 68. In the locked state, which is shown in FIG. 2, the locking pin 68 is engaged with a corresponding recess 70 in the first flange 40, so as to warrant a form-locked transmission of a torque between the auxiliary flange 66 and the first flange 40 in the mechanically defined angular position. In the unlocked state, the locking pin 68 is disengaged from the first flange 40, so as to allow a free relative rotation between the first flange 40 and the auxiliary flange 66. As the axis of the override bearing 60 is substantially coaxial to the axis of the joint bearing 46, the operator has the impression that the motorized rotary joint member 24 can now freely rotate about its pivot axis 26, despite the fact that it is blocked because of the afore-mentioned high reduction ratio in the drive means 50, 52. It will be noted that the amplitude of free pivot movement is normally limited by limit stops to an angle of less than +/-180° measured from the mechanically defined angular position.

In the robotic arm 16, the auxiliary flange 66 is e.g. connected to the support base 18 or to an arm member 22. The second flange 42 is connected to another arm member 22. If the motor unit 52 is stopped and locked prior to switching the rotational release unit 36 into its unlocked state, the arm member 22 connected to the second flange 42 can be manually pivoted out of a specific angular position, and can thereafter be easily brought back into said specific angular position, by manually pivoting it back, until the locking pin 68 engages again with the recess 70 in the first flange 40. Thus it is possible to pivot the arm member 22 manually out of a preset angular position, for example for allowing better access to the patient or to a specific body part of the patient, and then to pivot it manually back again into the pre-set angular position with great angular accuracy. It will be noted that the angular repositioning accuracy is the better, the greater the distance D between the locking pin 68 and the pivot axis 26 is. Assuming for example that this distance D is 300 mm, a play of 0.05 mm of the locking pin 68 in the recess 70 results in an angular play of less than 0.01°. The distance D will preferably greater than 150 mm.

If the embodiment of FIG. 2 is used for the rotary joint member 24₁ in FIG. 1, the auxiliary flange 66 is connected to the support base 18, and the second flange 42 is connected to the first arm member 22₁. In other words, the rotational release unit 36 is arranged between the support base 18 and the motorized rotary joint member 24. If the rotation locking mechanism 64 is switched into its unlocked state, the first arm member 22₁, can be freely rotated by hand about the pivot axis 26₁.

If the embodiment of FIG. 2 is for example used for the rotary joint member 24₂ in FIG. 1, the auxiliary flange 66 is preferably connected to the first arm member 22₁, and the second flange 42 is connected to the second arm member 22₂. In other words, the rotational release unit 36 is arranged between the first arm member 22₁ and the motorized rotary joint member 24. If the rotation locking mechanism 64 is switched into its unlocked state, the second arm member 22₂, can be freely rotated by hand about the pivot axis 26₂.

FIG. 3 schematically illustrates the motorized rotary joint member 24 associated with a rotational release unit 36', comprising an override bearing 60' and an auxiliary flange 66'. This embodiment, in which the motorized rotary joint member 24' is identical to that of FIG. 2, distinguishes over the embodiment of FIG. 2 mainly in that the override bearing 60' now connects the auxiliary flange 66' to the second flange 42 of the motorized rotary joint member 24. It will be noted that the joint bearing 46 and the override bearing 60 are, in this embodiment, located very closely together, which provides constructional advantages in many cases. Just as for the embodiment of FIG. 2, as the axis of the override bearing 60' is substantially coaxial to the axis of the joint bearing 46', one has the impression that-in the unlocked state of the rotational release unit 36'-the motorized rotary joint member 24 can freely rotate about its pivot axis 26, despite the fact that it is indeed blocked because of the aforementioned high reduction ratio in the drive means. It will be noted that the embodiment of FIG. 3 also warrants a similar repositioning accuracy as the embodiment of FIG. 2.

If the embodiment of FIG. 3 is used for the rotary joint member 24₁ in FIG. 1, the auxiliary flange 66' is connected to the second arm member 22₂, and the first flange 40 is connected to the support base 18. If it is used for the rotary joint member 24₂, the auxiliary flange 66' is connected to the second arm member 22₂, and the first flange 40 is connected to the first arm member 22₁.

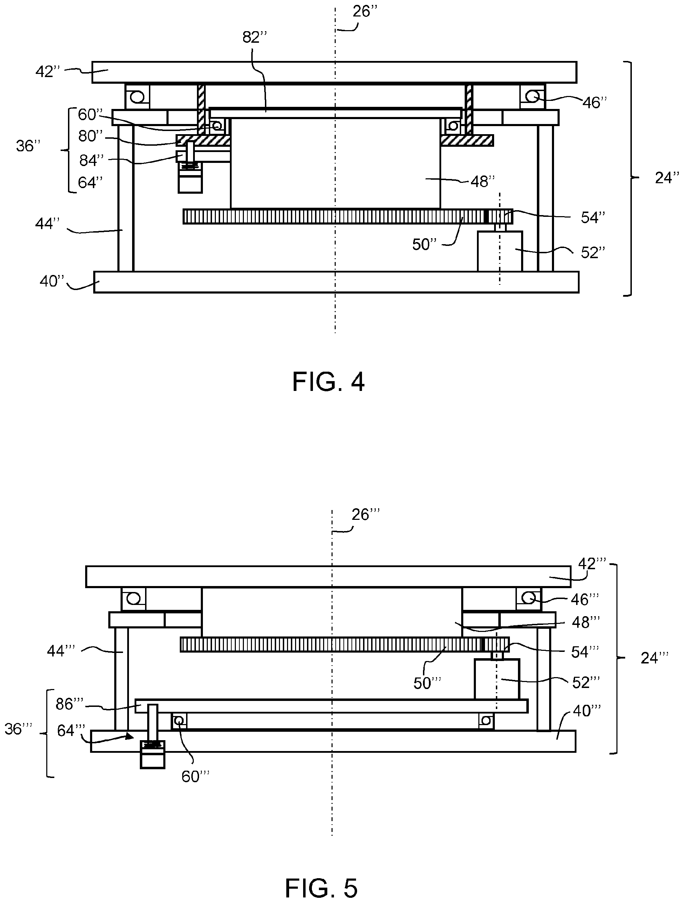

FIG. 4 shows a third embodiment of a motorized rotary joint member 24" associated with a rotational release unit 36", which is now integrated in the motorized rotary joint member 24". More particularly, the rotational release unit 36" is mounted between the second flange 42" and the annular drive gear 50". In a preferred embodiment, the override bearing 60" of the rotational release unit 36" is e.g. mounted on a flange 80" that is fixed to the second flange 42", so that the axis of rotation of the override bearing 60" is coaxial to the joint bearing 46". The tubular drive shaft 48" bearing the annular drive gear 50" comprises a flange 82", by means of which it is supported by the override bearing 60". The rotational release unit 36" further comprises a rotation locking mechanism 64" that is mounted e.g. on a flange 84" of the tubular drive shaft 48" (alternatively, the rotation locking mechanism 64" can also be mounted on the flange 80" fixed to the second flange 42"). It follows, that if the rotation locking mechanism 64" is in its locked state, it locks the tubular drive shaft 48" with the annular drive gear 50" in rotation relatively to the second flange 42", so that the motor unit 52" can rotate the second flange 42" about the pivot axis 26". If the rotation locking mechanism 64" is switched into its unlocked state, the second flange 42" can freely rotate about the coaxial axes of the override bearing 60" and the joint bearing 46", whereas the annular drive gear 50" is blocked by the motor unit 52".

The first flange 40" is e.g. connected to the support base 18 or to an arm member 22. The second flange 42" is generally connected to another arm member 22. If the motor unit 52" is stopped and locked prior to switching the rotational release unit 36" into its unlocked state, the arm member 22 connected to the second flange 42" can be manually pivoted about the pivot axis 26" out of a specific angular position, and can thereafter be easily brought back into said specific angular position, by manually pivoting it back, until the locking pin engages again with the recess in the flange 80". Consequently, after a temporary unlocking of the release unit 36", the embodiment of FIG. 4 achieves substantially the same repositioning accuracy as the embodiments of FIG. 2 and 3.

FIG. 5 shows a fourth embodiment of a motorized rotary joint member 24"' associated with a rotational release unit 36"', which is also integrated in the motorized rotary joint member 24"'. More particularly, the rotational release unit 36'" now includes an override bearing 60'" that is supported on the first flange 40'" and that supports the motor unit 52'" via a motor support flange 86"'. It follows that, if the rotation locking mechanism 64'" is switched into is unlocked state, the second flange 42'" can be freely rotated, together with the annular drive gear 50"', the motor unit 52'" (whose pinion 54'" is blocked in rotation) and the motor support flange 86"'. The first flange 40'" will however remain unaffected by this manual rotation of the second flange 42"'. Also the embodiment of FIG. 5 achieves, after a temporary release, substantially the same repositioning accuracy as the embodiments of FIG. 2 and 3.

It will be noted that the override bearings 60" and 60'" are generally less expensive than the override bearings 60 and 60', because the load constraints are less demanding. Indeed, whereas the override bearings 60 and 60' have to be dimensioned essentially for the same loads as the joint bearing 46, the override bearing 60" in FIG. 4 has to support only the tubular drive shaft 48" with the annular drive gear 50", and the override bearing 60'" in FIG. 5 has to support only the motor unit 52"'. However, the embodiments of FIG. 4 and 5 require a relatively precise alignment of the axes of rotation of the override bearing 60", 60'" with the joint bearing 46", 46"', whereas in the embodiments of FIG. 2 and 3, there are no such precise alignment constraints for the axes of rotation of the override bearing 60, 60' with the joint bearing 46, 46'. In the embodiments of FIG. 2 and 3, alignment constraints for these axes of rotation are only imposed by the design of the rotary joints in the outer casing of the robotic arm 16. Consequently, in the embodiments of FIG. 2 and 3, alignment constraints for the axes of rotation of the joint bearing and the override bearing may be reduced and even be entirely eliminated by an adequate design of the rotary joints in the outer casing of the robotic arm 16.

The joint bearings 36, 36', 36", 36'" and the override bearings will normally be rolling contact bearings 60, 60', 60", 60'" selected in function of the specific construction and operating conditions. It will further be understood that the mechanical structures shown in FIG. 2-5 have been very much simplified in order to better show the basic concepts underlying the present invention. In practice, the motorized rotary joint member 24, 24", 24'" will e.g. contain more than one joint bearing 46, 46", 46"'. Furthermore, the arrangement and mounting of the bearings 46, 46", 46'" has to be properly designed, duly considering design loads, bearing torques, dimensions and materials, required alignment and rotation precision etc.. Same applies to the rotational release units 36, 36', 36", 36'" and to the override bearings 60, 60', 60", 60"'.

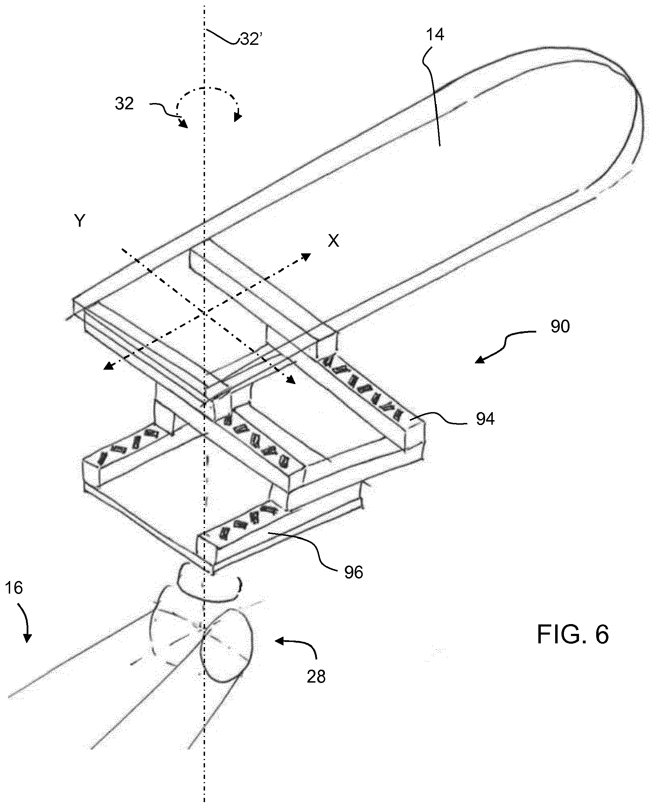

FIG.6 shows a translational release unit 90 connected between the orientation mechanism 28 (the robotic wrist 28) and the patient support table 14. The translational release unit 90 basically comprises an XY translation mechanism providing two translational degrees of freedom. Each degree of freedom is for example embodied by a linear stage 94, 96, comprising in a known manner a platform and a base, joined by some form of guide or linear bearing, in such a way that the platform is restricted to guided linear motion with respect to the base. The platform of the linear stage 94 supports the base of the linear stage 96, and the platform of the linear stage 96 supports the patient support table 14, so as to form two translational degrees of freedom that are perpendicular to one another.

The patient support table 14 is borne by the XY translation mechanism, so that its X-axis extends parallel to the length of the patient support table 14, and its Y-axis extends parallel to the width of the patient support table 14. Both linear stages 94, 96 are preferably free-moving, i.e. they do not include any mechanism or motor for moving the platform relative to the base. Movement of the patient support table 14 is achieved by manually pushing or pulling the patient support table 14.

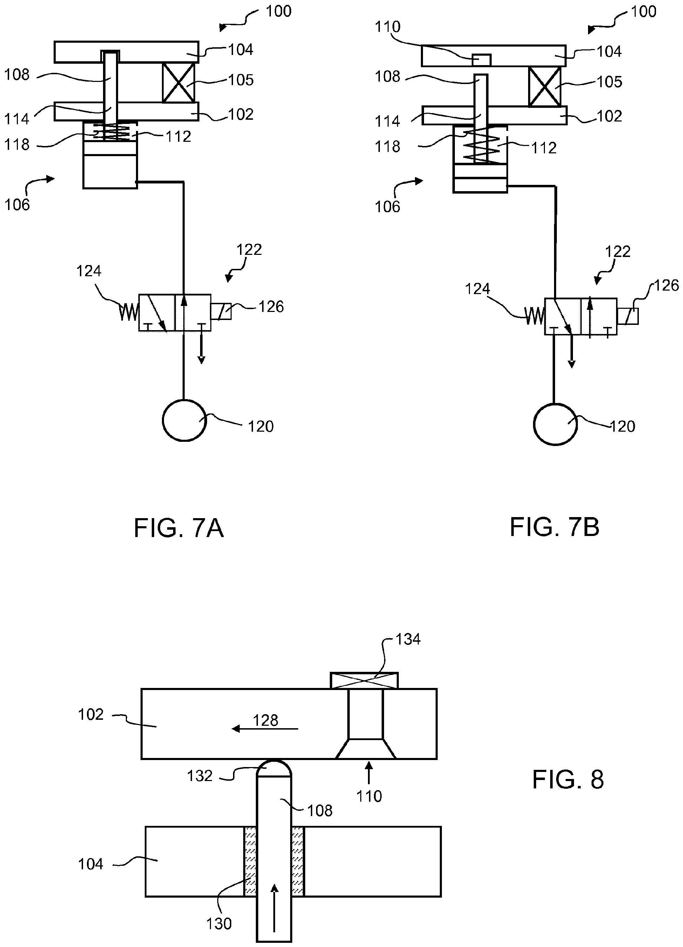

A translation locking mechanism (not seen in FIG. 6) cooperates with the XY translation mechanism , wherein it is switchable between a locked state, in which it locks the two linear stages 94, 96, and an unlocked state, in which the two linear stages 94, and 96 are unlocked (see also the description of FIG. 7A, 7B and 8). If the two linear stages 94 and 96 are unlocked, they allow a free planar translation movement of the patient support table 14 parallel to a plane that is perpendicular to the axis 32' of the top rotation angle 32 of the orientation mechanism (or robotic wrist). In other words, an operator may push or pull the patient support table 14 according to any direction perpendicular to the axis 32' of the top rotation angle 30. When the two linear stages 94, 96 are locked, they are both centred, preferably in a form-locked manner, on the axis 32' of the top rotation angle 32. With regard to this centred position, the XY translation mechanism provides a degree of freedom of +/x according to the X-axis and of +/- y according to the Y-axis, wherein the absolute values of x and y are preferably in the range of 300 mm to 800 mm. Preferably, each linear stage 94, 96 further includes a damper or brake for slowing down a gravity caused motion of the patient support means 14, if the translation locking mechanism is switched from is locked state in its unlocked state.

FIG. 7A and 7B are schematic diagrams further illustrating a locking mechanism 100 that may be used for a rotational release unit 36 or a translational release unit 90 as described hereinbefore. FIG. 7A shows the locking mechanism 100 in its locked status, and FIG. 7B in its unlocked status. This locking mechanism 100 is mounted between two flanges 102 and 104, which are mechanically interconnected either by a rotating bearing means, in case of a rotational release unit, or by a linear bearing means, in case of a translational release unit. In FIG. 7A and 7B, this rotating bearing means or linear bearing means is schematically represented by a crossed box 105, which generically stands for a relative movement bearing means.

The locking mechanism 100 shown in FIG. 7A and 7B comprises a linear actuator 106 bearing a locking pin 108. In the locked status, the locking pin 108 engages a recess 110 in the second flange 104, thereby locking the two flanges 102 and 104 in rotation or in translation, to warrant a form-locked transmission of a torque or a force between them.

The linear actuator 106 shown in FIG. 7A and 7B is more particularly a pneumatic cylinder, including a cylinder chamber 112, a piston rod 114 bearing the locking pin 108, and a return spring 118. The return spring 118 retracts the piston rod 114 into the cylinder chamber 112, when the latter is vented. Pressurizing the cylinder chamber 112 moves the piston rod 114 out of the cylinder chamber 112 and compresses the return spring 118. The pneumatic cylinder 106 is controlled by a control valve 122, schematically represented by a conventional graphic symbol. This control valve 122 comprises for example at least three ports and two valve positions. In the first valve position (shown in FIG. 7B), the first port is closed and the second port is internally connected to the third port. In the second valve position (shown in FIG. 7A), the first port is internally connected to the third port, and the second port is closed. A valve spring 124 urges the valve 122 into its first position, i.e. the rest position. A valve actuator 126 urges, if powered, the valve 122 into the second position. The valve actuator 126 is preferably connected to an uninterruptible power supply (not shown), i.e. a power supply with battery backup. When the connection between the valve actuator 126 and the uninterruptible power supply is interrupted, for example by pushing a release button (or preferably two release buttons mounted in parallel), the valve spring 124 urges the valve 122 into its first position.

Externally, the first port of the valve 122 is connected to a pressurized air source 120, its second port is vented (i.e. connected to atmosphere) and the third port is connected to the cylinder chamber 112. Consequently, when the valve 122 is in its first position (see FIG. 7B), the cylinder chamber 112 is vented, and when the valve 122 is in its second position (see FIG. 7A), the cylinder chamber 112 is pressurized.

Instead of using such a pneumatic cylinder as actuator for the locking pin 108, one may also use a linear drive that is hydraulically or electrically powered. Furthermore, instead of using a linear actuator 106 with a locking pin 108 axially engaged into a recess 110, one may also use a pivoting mechanism that is capable of pivoting a locking member, between a locked-position, in which it engages a cooperating locking means on the second flange 104, to provide a form-locked force transmission in the direction of relative movement of the two flanges 102, 104. However, the relatively simple, cost effective and reliable solution with the pneumatic cylinder 106 (or possibly another linear drive), the axially actuated locking pin 108 and the recess 110 is generally preferred.

In case of an XY translation mechanism, as described hereinbefore, each linear stage 94, 96 advantageously has its own locking mechanism 100. For example: the linear actuator 106 is fixed to an element of the base (which forms the first flange 102) and the locking pin 108 engages a recess in an element of the platform (which forms the second flange 104).

As long as the linear actuator 106 is powered, the locking pin 108 remains in the recess 110, providing a form-locked coupling between the two flanges 102 and 104. If the linear actuator 106 is unpowered, the return spring 118 (or another passive element) withdraws the locking pin 108 from the recess 110, thereby opening the coupling between the two flanges 102 and 104.

To re-establish a form-locked coupling between the two flanges 102 and 104, the linear actuator 106 is powered (i.e. the pneumatic cylinder is e.g. pressurized) to press the locking pin 108 with a front surface 132 against the surface of the second flange 104 into which the recess 110 opens. FIG. 8 shows the locking pin 108 in this position (the linear actuator 106 itself is not shown in FIG. 8, but his action is indicated by an arrow). By manually moving the flange 104 relatively to the flange 102 in the direction of the arrow 128, the recess 110 can be brought in alignment with the locking pin 108. To reduce friction between the front surface 132 of the locking pin 108 and the second flange 104, this front surface 132 advantageously has the form of a spherical dome and/or is advantageously coated with a friction reducing material. Alternatively, the front surface 132 of the locking pin 108 may also include a rolling ball, to achieve a rolling contact between the front surface 132 of the locking pin 108 and the second flange 104. The second flange 104 is provided with contact path having a surface quality adapted for a sliding contact, respectively a rolling contact with the front surface 132. When the locking pin 108 is aligned with the recess 110, the linear actuator 106 presses the locking pin 108 into the recess 110. To facilitate alignment of the locking pin 108 and the recess 110, the recess 110 advantageously has a cone-shaped opening, as shown in FIG. 8.

It will be understood that the first flange 102 only has to bear the linear actuator 106. It may consequently have a relatively small extension in the direction of the relative movement of the two flanges 102, 104. The second flange 104 not only has to bear the recess for receiving 108 the locking pin 108. It also has to form the (circular or linear) contact path for the front surface 132 of the locking pin 108. Its minimum extension in the direction of the relative movement of the two flanges 102, 104 is consequently determined by the length of this contact path, i.e. the extent of free relative movement the rotational release unit 36 or the translational release unit 90 shall provide.

In case of a rotational movement, the flange 104 need not necessarily be planar annular flange (as shown in the drawings) or an angular segment of such a planar annular flange. It may also be a cylindrical flange or a segment of such a cylindrical flange. In case of a cylindrical flange 104, the longitudinal axis of the locking pin 108 will be perpendicular to the axis of the rotational movement. (In the embodiments shown in the drawings, the longitudinal axis of the locking pin 108 is parallel to the axis of the rotational movement).

Reference number 134 points to a detector that is mounted in the recess 110 to detect that the locking pin 108 is in proper engagement with the recess 110. This detector 134 may e.g. be a pressure sensitive switch that is capable of monitoring an axial contact pressure of the locking pin 108 in the recess 110. A decrease of this axial contact pressure below a pre-set pressure may then trigger an alarm and/or be incorporated a security interlocking system of the positioning device 10 and/or of the medical equipment. It will be appreciated that monitoring the axial contact pressure of the locking pin 108 in the recess 110, allows to detect prior to the unlocking of the release unit that such unlocking may take place.

As further seen in FIG. 8, the locking pin 108 (or the piston rod 114 shown in FIG. 7A & 7B) is preferably guided (at least perpendicularly to the direction of movement that has to be locked) in a guide bushing 130 of the first flange 102, to avoid that its actuator 106 is subjected to important forces, when the locking pin 108 transfers a torque or a force from the first flange 102 to the second flange 104.

It will further be noted that the fit between the locking pin 108 and the recess 110 in the direction of the movement that has to be locked (i.e.: in case of a rotational movement locking, the direction tangential to the trajectory of the locking pin 108; and in case of a linear movement locking, the direction parallel to the respective X-axis or Y-axis) will strongly influence the positional accuracy of the repositioning. Consequently, whereas the fit between the locking pin 108 and the recess 110 in the direction of movement shall be relatively small (e.g. smaller than 1 mm, and preferably smaller than 0.1 mm), there may be an important clearance in the direction perpendicular to force transmission (i.e. in FIG. 8, in the direction perpendicular to the sheet). This important clearance perpendicular to force transmission makes the introduction of the locking pin 108 into the recess 110 easier.

Instead of using a cylindrical locking pin 108 (as shown in FIG. 8), it is also possible to use a tapered locking pin received in a tapered guide hole (similar to a machine tapers used for securing cutting bits and other accessories to a machine tool's spindle, as for example a so-called Morse taper system or another known taper system). Such a taper system may be preferred because of its auto-centring function, wherein it is generally preferable to limit the auto-centring function in the direction of the rotational or translational degree of freedom to be blocked.

The switching of the rotation or translation locking mechanism from the locked state into the unlocked state advantageously takes place according to the "two hands principle", i.e. the operator has to use his two hands to simultaneously push two release buttons to trigger this switching. These release buttons are preferably arranged close to the rotational release unit, respectively close to the translational release unit with whom they are associated. Alternatively or additionally, the device may include release buttons simultaneously releasing all motorized rotational degrees of freedom, or simultaneously releasing all motorized rotational degrees of freedom with a vertical pivot axis.

**List of Reference Signs**

| | | | |
|---|---|---|---|
| 10 | device for supporting and positioning a patient | 68 | locking member or pin |
| | | 70 | recess |
| 12 | nozzle of medical equipment | 80" | flange of 36" |
| 14 | patient support means | 82" | flange of 36" |
| 16 | robotic arm | 84"' | flange of 36'" |
| 18 | support base | 90 | translational release unit |
| 20 | floor | 94 | linear stage (X-axis) |
| 22 | support member (arm member); | 96 | linear stage (Y-axis) |
| 24 | motorized rotary joint member | 100 | locking means |
| 26 | pivot axis | 102 | first flange of 100 |
| 28 | orientation mechanism (robotic wrist) | 104 | second flange of 100 |
| | | 105 | relative movement bearing means |
| 30 | pitch angle | 106 | linear actuator/ pneumatic cylinder |
| 32 | top rotation angle | 108 | locking pin |
| 34 | roll angle | 110 | recess |
| 36 | rotational release unit | 112 | cylinder chamber |
| 40 | first flange of 24 | 114 | piston rod |
| 42 | second flange of 24 | 118 | spring |
| 44 | spacing structure | 122 | control valve |
| 46 | joint bearing | 120 | pressurized air source |
| 48 | tubular drive shaft | 124 | valve spring |
| 50 | annular drive gear | 126 | valve actuator |
| 52 | motor unit | 128 | arrow, indicating the direction of movement |
| 54 | pinion | | |
| 60 | override bearing | 130 | guide bushing |
| 64 | rotation locking mechanism | 132 | front surface of 108 |
| 66 | auxiliary flange | 134 | detector |

## Claims

1. A device for supporting and positioning a patient in a medical equipment, comprising:
a patient support means (14); and
a robotic arm (16) supporting said patient support means;
a robotic wrist (28) with at least two motorized rotational degrees of freedom, said robotic wrist being borne by said robotic arm and bearing said patient support means;
**characterized by:**
a translational release unit connected between said robotic wrist and said patient support means, said translational release unit including:
an XY translation mechanism providing two translational degrees of freedom; and
a translation locking mechanism (64) cooperating with said XY translation
mechanism, said translation locking mechanism being switchable between a locked state, in which it locks said two translational degrees of freedom of said XY translation mechanism in a mechanically defined position, and an unlocked state, in which said two translational degrees of freedom are unlocked.

2. The device as claimed in claim 1, wherein:
each degree of freedom is embodied by a linear stage, comprising a platform and a base, which are joined by a linear guide or bearing means, in such a way that said platform is restricted to guided linear motion with respect to said base.

3. The device as claimed in claim 2, wherein:
each stage comprises a separate translation locking mechanism.

4. The device as claimed in claim 1, 2 or 3, wherein:
said translation locking mechanism comprises a locking pin providing in its locked state a form-locked locking in said mechanically defined position.

5. The device as claimed in claim 4, wherein:
said locking pin is a tapered pin which is capable of engaging a tapered guide hole, so as to provide, in said mechanically defined position, an auto-centring function in the direction of the translational degree of freedom to be blocked.

6. The device as claimed in any one of the preceding claims, wherein:
said rotation locking mechanism has to be powered to switch into said locked state and, if it is unpowered, it switches into said unlocked state.

7. The device as claimed in claim 4, wherein:
said translation locking mechanism includes a linear drive for driving a locking member in a locking position, said linear drive being electrically, hydraulically or pneumatically powered; and
said linear drive includes a passive element, preferably a spring, for urging said locking member out of said locking position, if said linear drive is unpowered.

8. The device as claimed in claim 6 or 7, wherein said rotation locking mechanism includes:
a pneumatic cylinder with a cylinder chamber, a piston, a piston rod and a return spring, said return spring retracting said piston rod into said cylinder chamber when the latter is vented; and
a control valve connected to said cylinder chamber, said control valve connecting said cylinder chamber to a pressure source when it is powered and venting it when it is unpowered.

9. The device as claimed in any one of the preceding claims, wherein:
the switching of the translation locking mechanism from the locked state into the unlocked state is triggered by simultaneously pushing two release buttons, which are preferably arranged so that an operator has to use his two hands to trigger this switching.

10. The device as claimed in any one of the preceding claims, wherein:
said robotic wrist includes three motorized rotational degrees of freedom, for controlling: a pitch angle, which allows a back and forward tilting of said patient support table; a top rotation angle, which allows a planar swivelling of said patient support table, and a roll angle, which allows a side to side pivoting of said patient support table; and
the two translational degrees of freedom are parallel to a plane that is perpendicular to the axis of said top rotation angle.

11. The device as claimed in claim 10, wherein:
said XY translation mechanism is centred on the axis of the top rotation angle, when it is in its locked state.

12. The device as claimed in claim 11, wherein:
with regard to its centred position, the XY translation mechanism provides a degree of freedom of +/- x according to the X-axis and of +/- y according to the Y axis, wherein the absolute values of x and y are in the range of 300 mm to 800 mm.

13. The device as claimed in any one of the preceding claims, wherein:
said robotic arm includes at least one motorized rotary joint member for positioning the patient support means by means of a motorized pivoting motion about a pivot axis; and
a rotational release unit associated with said motorized rotary joint member, said rotational release unit comprising:
an override bearing arranged adjacent to or in said in said motorized rotary joint member so as to be substantially coaxial with said pivot axis, and to allow a free pivoting motion of said positioning mechanism about said pivot axis; and
a rotation locking mechanism cooperating with said override bearing, said rotation locking mechanism being switchable between a locked state, in which it locks said override bearing in a mechanically defined angular position, and an unlocked state, in which said override bearing is unlocked and said positioning mechanism can freely pivot about said pivot axis.

14. The device as claimed in claim 13, wherein:
said robotic arm comprises two motorized rotary joint members defining two substantially vertical pivot axes, wherein each of said two motorized rotary joint members has associated therewith a rotational release unit as defined in claim 13.

15. The device as claimed claim 13 or 14, wherein:
said robotic arm comprises a motorized rotary joint member that has a substantially horizontal pivot axis; and
a damper or brake is integrated in said rotational release unit for slowing down a gravity caused pivoting motion about said substantially horizontal pivot axis, when said rotation locking mechanism of said rotational release unit is switched from said locked state into said unlocked state.

## Patentansprüche

1. Vorrichtung zum Tragen und Positionieren eines Patienten in einer medizinischen Einrichtung, umfassend:
ein Patiententragmittel (14); und
einen Roboterarm (16), der das Patiententragmittel trägt;
ein Roboterhandgelenk (28) mit mindestens zwei motorisierten Rotationsfreiheitsgraden, wobei das Roboterhandgelenk durch den Roboterarm getragen wird und das Patiententragmittel trägt;
**gekennzeichnet durch**:
eine Translationsfreigabeeinheit, die zwischen dem Roboterhandgelenk und dem Patiententragmittel angeschlossen ist, wobei die Translationsfreigabeeinheit umfasst:
einen XY-Translationsmechanismus, der zwei Translationsfreiheitsgrade bereitstellt; und
einen Translationssperrmechanismus (64), der mit dem XY-Translationsmechanismus zusammenwirkt, wobei der Translationssperrmechanismus zwischen einem gesperrten Zustand, in welchem er die beiden Translationsfreiheitsgrade des XY-Translationsmechanismus in einer mechanisch definierten Position sperrt, und einem entsperrten Zustand, in welchem die beiden Translationsfreiheitsgrade entsperrt sind, gewechselt werden kann.

2. Vorrichtung nach Anspruch 1, wobei:
jeder Freiheitsgrad durch einen Lineartisch realisiert ist, der eine Plattform und eine Basis umfasst, die durch eine Linearführung oder Linearlagermittel derart verbunden sind, dass die Plattform auf geführte lineare Bewegung in Bezug auf die Basis beschränkt ist.

3. Vorrichtung nach Anspruch 2, wobei:
jeder Tisch einen separaten Translationssperrmechanismus aufweist.

4. Vorrichtung nach Anspruch 1, 2 oder 3, wobei:
der Translationssperrmechanismus einen Sperrstift umfasst, der in seinem gesperrten Zustand eine formschlüssige Sperre in der mechanisch definierten Position bereitstellt.

5. Vorrichtung nach Anspruch 4, wobei:
der Sperrstift ein Keilstift ist, der in ein konisches Führungsloch eingreifen kann, um in der mechanisch definierten Position eine selbstzentrierende Funktion in der Richtung des zu blockierenden Translationsfreiheitsgrades bereitzustellen.

6. Vorrichtung nach einem der vorhergehenden Ansprüche, wobei:
der Rotationssperrmechanismus angetrieben werden muss, um in den gesperrten Zustand zu wechseln, und in den entsperrten Zustand wechselt, wenn er nicht angetrieben wird.

7. Vorrichtung nach Anspruch 4, wobei:
der Translationssperrmechanismus einen Linearantrieb zum Antreiben eines Sperrelements in einer Sperrposition umfasst, wobei der Linearantrieb elektrisch, hydraulisch oder pneumatisch betrieben ist; und
der Linearantrieb ein passives Element, vorzugsweise eine Feder, zum Treiben des Sperrelements aus der Sperrposition umfasst, wenn der Linearantrieb nicht angetrieben wird.

8. Vorrichtung nach Anspruch 6 oder 7, wobei der Rotationssperrmechanismus umfasst:
einen pneumatischen Zylinder mit einer Zylinderkammer, einem Kolben, einer Kolbenstange und einer Rückstellfeder, wobei die Rückstellfeder die Kolbenstange in die Zylinderkammer zurückzieht, wenn Letztere gelüftet wird; und
ein Regelventil, das mit der Zylinderkammer verbunden ist, wobei das Regelventil die Zylinderkammer mit einer Druckquelle verbindet, wenn es angetrieben wird, und sie lüftet, wenn es nicht angetrieben wird.

9. Vorrichtung nach einem der vorhergehende Ansprüche, wobei:
das Wechseln des Translationssperrmechanismus vom gesperrten Zustand in den entsperrten Zustand durch gleichzeitiges Drücken zweier Freigabetasten ausgelöst wird, die vorzugsweise so angeordnet sind, dass ein Bediener seine beiden Hände zum Auslösen dieses Wechselns verwenden muss.

10. Vorrichtung nach einem der vorhergehenden Ansprüche, wobei:
das Roboterhandgelenk drei motorisierte Rotationsfreiheitsgrade umfasst zum Steuern: eines Nickwinkels zum Ermöglichen von Vor- und Rückwärtsneigung des Patiententragtisches; eines Top-Rotationswinkels zum Ermöglichen planarer Schwenkung des Patiententragtisches und eines Wankwinkels zum Ermöglichen von Drehung des Patiententragtisches von Seite zu Seite; und
die zwei Translationsfreiheitsgrade parallel zu einer Ebene sind, die senkrecht auf die Achse des Top-Rotationswinkel ist.

11. Vorrichtung nach Anspruch 10, wobei:
der XY-Translationsmechanismus auf der Achse des Top-Rotationswinkels zentriert ist, wenn er in seinem gesperrten Zustand ist.

12. Vorrichtung nach Anspruch 11, wobei:
der XY-Translationsmechanismus in Bezug auf seine zentrierte Position einen Freiheitsgrad von +/- gemäß der X-Achse und +/- gemäß der Y-Achse bereitstellt, wobei die absoluten Werte von x und y im Bereich von 300 mm bis 800 mm liegen.

13. Vorrichtung nach einem der vorhergehenden Ansprüche, wobei:
der Roboterarm mindestens ein motorisiertes Drehverbindungselement zum Positionieren des Patiententragmittels durch eine motorisierte Drehbewegung um eine Drehachse umfasst; und
eine Rotationsfreigabeeinheit mit dem motorisierten Drehverbindungselement assoziiert ist, wobei die Rotationsfreigabeeinheit umfasst:
ein Freilauflager, das benachbart zu oder in dem motorisierten Drehverbindungselement angeordnet ist, um im Wesentlichen koaxial mit der Drehachse zu sein und freie Drehbewegung des Positioniermechanismus um die Drehachse zu ermöglichen; und
einen Rotationssperrmechanismus, der mit dem Freilauflager zusammenwirkt, wobei der Rotationssperrmechanismus zwischen einem gesperrten Zustand, in welchem er das Freilauflager in einer mechanisch definierten Winkelposition sperrt, und einem entsperrten Zustand, in welchem das Freilauflager entsperrt ist und sich der Positioniermechanismus frei um die Drehachse drehen kann, gewechselt werden kann.

14. Vorrichtung nach Anspruch 13, wobei:
der Roboterarm zwei motorisierte Drehverbindungselemente umfasst, die zwei im Wesentlichen vertikale Drehachsen definieren, wobei jedes der zwei motorisierten Drehverbindungselemente eine Rotationsfreigabeeinheit nach Anspruch 13 damit assoziiert aufweist.

15. Vorrichtung nach Anspruch 13 oder 14, wobei:
der Roboterarm ein motorisiertes Drehverbindungselement umfasst, das eine im Wesentlichen horizontale Drehachse aufweist; und
ein Dämpfungselement oder eine Bremse zum Abschwächen einer durch Schwerkraft verursachten Drehbewegung um die im Wesentlichen horizontale Achse, wenn der Rotationssperrmechanismus der Rotationsfreigabeeinheit vom gesperrten Zustand in den entsperrten Zustand gewechselt wird, in die Rotationsfreigabeeinheit integriert ist.

## Revendications

1. Dispositif de support et de positionnement d'un patient dans un équipement médical, comprenant :
des moyens de support de patient (14) ; et
un bras robotisé (16) supportant lesdits moyens de support de patient ;
un poignet robotisé (28) avec au moins deux degrés de liberté de rotation motorisée, ledit poignet robotisé étant supporté par ledit bras robotisé et portant lesdits moyens de support de patient ;
**caractérisé par** :
une unité de libération translationnelle reliée entre ledit poignet robotisé et lesdits moyens de support de patient, ladite unité de libération translationnelle comprenant :
un mécanisme de translation XY garantissant deux degrés de liberté translationnelle ;
et
un mécanisme de blocage de translation (64) coopérant avec ledit mécanisme de translation XY, ledit mécanisme de blocage de translation pouvant basculer entre un état bloqué, dans lequel il bloque lesdits deux degrés de liberté translationnelle dudit mécanisme de translation XY dans une position mécaniquement définie, et un état débloqué, dans lequel lesdits deux degrés de liberté translationnelle sont débloqués.

2. Dispositif selon la revendication 1, dans lequel :
chaque degré de liberté est intégré à un étage linéaire, comprenant une plate-forme et une base, qui sont jointes par un guide linéaire ou des moyens de support, de sorte que ladite plate-forme soit limitée à un mouvement linéaire guidé par rapport à ladite base.

3. Dispositif selon la revendication 2, dans lequel :
chaque étage comprend un mécanisme de blocage de translation distinct.

4. Dispositif selon la revendication 1, 2 ou 3, dans lequel :
ledit mécanisme de blocage de translation comprend une goupille de verrouillage qui assure, dans son état bloqué, un blocage par forme dans ladite position mécaniquement définie.

5. Dispositif selon la revendication 4, dans lequel :
ladite goupille de verrouillage est une goupille pointue qui est capable d'engager un orifice de guide pointu, de façon à assurer, dans ladite position mécaniquement définie, une fonction d'auto-centrage dans la direction du degré de liberté translationnelle à bloquer.

6. Dispositif selon l'une quelconque des revendications précédentes, dans lequel :
ledit mécanisme de blocage de rotation doit être alimenté pour passer dans ledit état bloqué et, s'il n'est pas alimenté, passe audit état débloqué.

7. Dispositif selon la revendication 4, dans lequel :
ledit mécanisme de blocage de translation comprend un entraînement linéaire destiné à entraîner un élément de blocage dans une position de blocage, ledit entraînement linéaire étant alimenté de manière électrique, hydraulique ou pneumatique ; et
ledit entraînement linéaire comprend un élément passif, de préférence un ressort, destiné à faire sortir ledit élément de blocage de ladite position de blocage, si ledit entraînement linéaire n'est pas alimenté.

8. Dispositif selon la revendication 6 ou 7, dans lequel ledit mécanisme de blocage de rotation comprend :
un vérin pneumatique avec une chambre de vérin, un piston, une tige de piston et un ressort de rappel, ledit ressort de rappel rétractant ladite tige de piston dans ladite chambre de vérin lorsque cette dernière est évacuée ; et
une soupape de commande est reliée à ladite chambre de vérin, ladite soupape de commande reliant ladite chambre de vérin à une source de pression lorsqu'elle est alimentée, et l'évacuant lorsqu'elle n'est pas alimentée.

9. Dispositif selon l'une quelconque des revendications précédentes, dans lequel :
le passage du mécanisme de blocage de translation de l'état bloqué à l'état débloqué est déclenché en appuyant simultanément sur deux boutons de libération, qui sont de préférence disposés de sorte qu'un opérateur doive utiliser ses deux mains pour déclencher cette opération.

10. Dispositif selon l'une quelconque des revendications précédentes, dans lequel :
ledit poignet robotisé comprend trois degrés de liberté de rotation motorisée, pour contrôler : un angle de tangage, qui permet une inclinaison vers l'avant et vers l'arrière de ladite table de support de patient ; un angle de rotation supérieur, qui permet un pivotement planaire de ladite table de support de patient, et un angle de roulis, qui permet un pivotement latéral de ladite table de support de patient ; et
les deux degrés de liberté translationnelle sont parallèles à un plan qui est perpendiculaire à l'axe dudit angle de rotation supérieur.

11. Dispositif selon la revendication 10, dans lequel :
ledit mécanisme de translation XY est centré sur l'axe de l'angle de rotation supérieur, lorsqu'il se trouve dans son état bloqué.

12. Dispositif selon la revendication 11, dans lequel :
en ce qui concerne sa position centrée, le mécanisme de translation XY offre un degré de liberté de +/-x selon l'axe X, et de +/-y selon l'axe X, les valeurs absolues de x et y étant de l'ordre de 300 mm à 800 mm.

13. Dispositif selon l'une quelconque des revendications précédentes, dans lequel :
ledit bras robotisé comprend au moins un élément de jonction rotatif motorisé destiné à positionner les moyens de support de patient à l'aide d'un mouvement de pivotement motorisé autour d'un axe de pivotement ; et
une unité de libération de rotation associée audit élément de jonction rotatif motorisé, ladite unité de libération de rotation comprenant :
un palier de maintien adjacent à ou dans ledit élément de jonction rotatif motorisé de façon à être sensiblement coaxial avec ledit axe de pivotement, et à permettre un mouvement de pivotement libre dudit mécanisme de positionnement autour dudit axe de pivotement ; et
un mécanisme de blocage de rotation coopérant avec ledit palier de maintien, ledit mécanisme de blocage de rotation pouvant passer entre un état bloqué, dans lequel il bloque ledit palier de maintien dans une position angulaire mécaniquement définie, et un état débloqué, dans lequel ledit palier de maintien est débloqué et ledit mécanisme de pivotement peut pivoter librement autour dudit axe de pivotement.

14. Dispositif selon la revendication 13, dans lequel :
ledit bras robotisé comprend deux éléments de jonction rotatifs motorisés définissant deux axes de pivotement sensiblement verticaux, chaque desdits deux éléments de jonction rotatifs motorisés étant associé à une unité de libération de rotation selon la revendication 13.

15. Dispositif selon la revendication 13 ou 14, dans lequel :
ledit bras robotisé comprend un élément de jonction rotatif motorisé qui possède un axe de pivotement sensiblement horizontal ; et
un amortisseur ou un frein est intégré à ladite unité de libération de rotation pour ralentir une gravité provoquée par le mouvement de pivotement autour dudit axe de pivotement sensiblement horizontal, lorsque ledit mécanisme de blocage de rotation de ladite unité de libération de rotation passe dudit état bloqué audit état débloqué.
